# EUROPEAN PATENT SPECIFICATION

(11) **EP 2 442 673 B1**
(45) Date of publication and mention of the grant of the patent: **10.07.2013**
(21) Application number: 11705724.0
(22) Date of filing: 28.01.2011
(51) Int. Cl.: A23L 1/29, A23L 1/30, A23L 3/46, A61K 31/19

(54) **NUTRITIONAL POWDERS COMPRISING SPRAY DRIED HMB**
NAHRUNGSPULVER MIT SPRÜHGETROCKNETEM HMB
POUDRES NUTRITIONELLES COMPRENANT DU BETA-HYDROXY-BETA-METHYLBUTYRATE SECHE PAR PULVERISATION

(30) Priority: 29.01.2010 US 299632 P
(43) Date of publication of application: 25.04.2012
(73) Proprietor: Abbott Laboratories, Abbott Park, IL 60064 (US)
(72) Inventor: HELMKE, Charles, R, Gahanna, Ohio 43230 (US)
(74) Representative: Modiano, Micaela Nadia
(86) International application number: PCT/US2011/022935
(87) International publication number: WO 2011/094549

(56) References cited:
- WO-A2-2007/075605
- US-A1- 2004 048 925
- US-B1- 6 291 525
- KREIDER R B ET AL: "EFFECT OF CALCIUM BETA-HYDROXY-BETA-METHYLBUTYRATE (HMB) SUPPLEMENTATION DURING RESISTANCE-TRAINING ON MARKERS OF CATABOLISM, BODY COMPOSITION AND STRENGTH", INTERNATIONAL JOURNAL OF SPORTS MEDICINE, THIEME, STUTTGART, DE, vol. 20, no. 8, 1 November 1999 (1999-11-01), pages 503-509, XP008037671, ISSN: 0172-4622, DOI: DOI:10.1055/S-1999-8835

## Description

### FIELD OF THE DISCLOSURE

The present disclosure relates to nutritional powders comprising spray dried beta-hydroxy-beta-methylbutyrate (HMB).

### BACKGROUND OF THE DISCLOSURE

Beta-hydroxy-beta-methylbutyrate (HMB) is a naturally occurring amino acid metabolite that is known for use in a variety of nutritional products and supplements. HMB is commonly used in such products to help build or maintain healthy muscle in selected individuals.

HMB is a metabolite of the essential amino acid leucine and has been shown to modulate protein turnover and inhibit proteolysis. In most individuals, muscle converts approximately 5% of available leucine to HMB, thus producing about 0.2 to 0.4 grams of HMB per day in a 70 kg male. In studies where various kinds of stress were induced in animals, HMB supplementation increased lean mass. Clinical studies also suggest that HMB has at least two functions in recovery from illness or injury including protection of lean mass from stress-related damage and enhancement of protein synthesis. It has been suggested that HMB may also be useful for enhancing immune function, reducing the incidence or severity of allergy or asthma, reducing total serum cholesterol and low density lipoprotein cholesterol, increasing the aerobic capacity of muscle, and other uses.

Since HMB is most often administered to individuals to support the development and maintenance of muscle mass and strength, many HMB products have been formulated with additional nutrients that may also be helpful in promoting healthy muscle. Some of these HMB products contain additional nutrients such as fat, carbohydrate, protein, vitamins, minerals and so forth. Calcium HMB is a commonly used form of HMB when formulated in oral nutritional products, which products include tablets, capsules, reconstitutable powders, and nutritional liquids and emulsions. Reconstitutable powders are particularly useful in this regard because such powders are often more shelf-stable than their liquid counterparts for extended periods even when formulated with multiple ingredients such as amino acids, carbohydrates, protein, and fat. These powders, however, often give off an undesirable aroma or odor, which is especially pronounced in the headspace of packaged product immediately after opening prior to use. Spray-dried HMB powder is for example discloses in US 2004 048925.

There is therefore a need for nutritional powders comprising HMB that are substantially free of undesirable aromas and off odors, especially when packaged and stored for extended periods.

### SUMMARY OF THE DISCLOSURE

One embodiment of the present disclosure is directed to a nutritional powder comprising HMB and at least one of protein, carbohydrate, and fat, wherein at least a portion of the HMB is spray dried in combination with at least a portion of at least one of the protein, carbohydrate and fat in the nutritional powder.

Another embodiment of the present disclosure is directed to a nutritional powder comprising from about 0.1% to about 8% HMB, from about 5% to about 70% protein, from about 10% to about 75% carbohydrate, and from about 20% to about 85% fat, all by weight of the nutritional powder, wherein least a portion of the HMB is spray dried with at least a portion of at least one of the protein, fat, and carbohydrate in the composition.

Another embodiment of present disclosure is directed to a method of preparing a nutritional powder comprising spray dried HMB. The method comprises preparing a liquid composition comprising HMB and at least one of protein, carbohydrate, and fat, and then spray drying the liquid composition to produce a nutritional powder having improved sensory properties

It has now also been found that the undesirable aromas or odors from nutritional powders comprising HMB can be minimized or eliminated by formulating the nutritional powders with spray dried HMB, wherein the HMB is spray dried with at least a portion of at least one of the protein, fat, and carbohydrate in the composition.

### DETAILED DESCRIPTION OF THE DISCLOSURE

The nutritional powders comprise spray dried HMB and other nutrients. These and other features of the nutritional powders, including methods of making such powders, as well as some of the many optional variations and additions thereof, are described in detail hereafter.

The term "HMB" as used herein, unless otherwise specified, refers to beta-hydroxy-beta-methylbutyrate (also referred to as beta-hydroxyl-3-methyl butyric acid, beta-hydroxy isovaleric acid) and sources thereof. All weights, percentages, and concentrations as used herein to characterize HMB are based on the weight of HMB, except that all weights, percentages, and concentrations as used herein to characterize calcium HMB are based on the weight of calcium HMB monohydrate, unless otherwise specified.

The term "nutritional powder" as used herein, unless otherwise specified, refers to nutritional powders comprising HMB and at least one of fat, protein, and carbohydrate, which are reconstitutable with an aqueous liquid, and which are suitable for oral administration to a human.

The terms "fat" and "oil" as used herein, unless otherwise specified, are used interchangeably to refer to lipid materials derived or processed from plants or animals.

All percentages, parts and ratios as used herein, are by weight of the total composition, unless otherwise specified. All such weights as they pertain to listed ingredients are based on the active level and, therefore, do not include solvents or byproducts that may be included in commercially available materials, unless otherwise specified.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The various embodiments of the nutritional powders of the present disclosure may also be substantially free of any optional or selected essential ingredient or feature described herein, provided that the remaining nutritional powder still contains all of the required ingredients or features as described herein. In this context, and unless otherwise specified, the term "substantially free" means that the selected nutritional powder contains less than a functional amount of the optional ingredient, typically less than about 0.5%, including less than about 0.1%, and also including zero percent, by weight of such optional or selected essential ingredient.

The nutritional powders and corresponding manufacturing methods of the present disclosure can comprise, consist of, or consist essentially of the essential elements of the disclosure as described herein, as well as any additional or optional element described herein or otherwise useful in nutritional powder formula applications.

### Product Form

The nutritional powders are typically in the form of flowable or substantially flowable particulate compositions, or at least particulate compositions that can easily be scooped and measured with a spoon or similar other device, wherein the compositions can easily be reconstituted by the intended user with a suitable aqueous liquid, typically water, to form a liquid nutritional formulation for immediate oral or enteral use. In this context, "immediate" use generally means within about 48 hours, most typically within about 24 hours, preferably right after reconstitution.

The nutritional powders may be formulated with sufficient kinds and amounts of nutrients so as to provide a sole, primary, or supplemental source of nutrition, or to provide a specialized nutritional powder for use in individuals afflicted with specific diseases or conditions. In one specific embodiment, the nutritional powder may be formulated for use with individuals for maintaining or building muscle mass.

The nutritional powders may be reconstituted with water prior to use to a caloric density tailored to the nutritional needs of the ultimate user, although in most instances the powders are reconstituted with water to form compositions comprising from about 100 to about 500 kcal/240 ml, including from about 150 to about 350 kcal/240 ml. and also including from about 200 to about 320 kcal/240 ml. The amount of calcium HMB in the reconstituted liquids most typically ranges from about 0.4 to about 3.0 g/240 ml, including from about 0.75 to about 2.0 g/240 ml, including about 1.5 g/240 ml.

Although the serving size for the reconstituted nutritional liquid can vary depending upon a number of variables, a typical serving size ranges from about 100 to about 300 ml, including from about 150 to about 250 ml, including from about 190 ml to about 240 ml.

### Macronutrients

The nutritional powders comprise at least one of fat, protein, and carbohydrate. Generally, any source of fat, protein, and carbohydrate that is known or otherwise suitable for use in nutritional products may also be suitable for use herein, provided that such macronutrients are also compatible with the essential elements of the nutritional powders as defined herein.

Although total concentrations or amounts of the fat, protein, and carbohydrate may vary depending upon the nutritional needs of the intended user, such concentrations or amounts most typically fall within one of the following embodied ranges, inclusive of any other essential fat, protein, and or carbohydrate ingredients as described herein.

Fat concentrations in the nutritional powders may range from 0 to about 99%, including from about 5% to about 75%, including from about 10% to about 35%, and also including from about 15% to about 20%, by weight of the nutritional powder.

Carbohydrate concentrations may range from 0 to about 99%, including from about 20% to about 90%, including from about 30% to about 80%, and also including from about 40% to about 70%, by weight of the nutritional powder.

Protein concentrations may range from 0 to about 90%, including from about 5% to about 75%, including from about 8% to about 50%, and also including from about 12% to about 30%, by weight of the nutritional powder.

The range or level of carbohydrate, fat, and protein in the nutritional powders may also be characterized in addition to or in the alternative as a percentage of total calories in the nutritional powder as set forth in the following table.

| Nutrient | Embodiment A (% of Total Calories) | Embodiment B (% of Total Calories) | Embodiment C (% of Total Calories) |
|---|---|---|---|
| Carbohydrate | 1-98 | 10-75 | 30-50 |
| Fat | 1-98 | 20-85 | 35-55 |
| Protein | 1-98 | 5-70 | 15-35 |

Non-limiting examples of suitable fats or sources thereof for use in the nutritional powders include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof.

Non-limiting examples of suitable carbohydrates or sources thereof for use in the nutritional powders include maltodextrin, hydrolyzed or modified starch or cornstarch, glucose polymers, corn syrup, corn syrup solids, rice-derived carbohydrates, glucose, fructose, lactose, high fructose corn syrup, honey, sugar alcohols (e.g., maltitol, erythritol, sorbitol), and combinations thereof.

Non-limiting examples of suitable protein or sources thereof for use in the nutritional powders include hydrolyzed, partially hydrolyzed or non-hydrolyzed proteins or protein sources, which may be derived from any known or otherwise suitable source such as milk (e.g., casein, whey), animal (e.g., meat, fish), cereal (e.g., rice, corn), vegetable (e.g., soy) or combinations thereof. Non-limiting examples of such proteins include milk protein isolates, milk protein concentrates as described herein, casein protein isolates, whey protein, sodium or calcium caseinates, whole cow's milk, partially or completely defatted milk, soy protein isolates, soy protein concentrates, and so forth.

### HMB

The HMB component of the nutritional powders may be obtained from any HMB source suitable for use in a nutritional product. Such sources include HMB as a free acid, a salt, an anhydrous or hydrated salt, an ester, a lactone, or other forms that otherwise provide a bioavailable form of HMB from the nutritional powder.

Non-limiting examples of suitable HMB sources include HMB salts, hydrated or anhydrous, of sodium, potassium, magnesium, chromium, calcium, or other non-toxic salt form. Calcium HMB is preferred, and is most typically formulated or otherwise obtained as calcium HMB monohydrate, a commercially available source of which is available from Technical Sourcing International (TSI) of Salt Lake City, Utah USA.

The total concentration of HMB in the nutritional powders may range up to about 10%, including from about 0.1 % to about 8%, and also including from about 0.2% to about 5.0%, and also including from about 0.3% to about 3%, and also including from about 0.4% to about 1.5%, by weight of the nutritional powder. Such concentrations may represent the concentration of HMB or the HMB source for use in the composition.

All or a portion of the HMB in the nutritional powder is in the form of spray dried HMB, as described herein.

### Vitamin D

The nutritional powders may further comprise vitamin D to help maintain healthy muscle in the targeted user. Vitamin D forms include Vitamin D2 (ergocalciferol) and Vitamin D3 (cholecalciferol) or other forms suitable for use in a nutritional product.

The amount of Vitamin D in the nutritional powder typically ranges up to about 1000 IU, more typically from about 10 to about 600 IU, and more typically from about 50 to 400 IU per serving size as described herein.

### Optional Ingredients

The nutritional powders may further comprise other optional ingredients that may modify the physical, chemical, hedonic or processing characteristics of the products or serve as pharmaceutical or additional nutritional components when used in the targeted population. Many such optional ingredients are known or otherwise suitable for use in other nutritional products and may also be used in the nutritional powders described herein, provided that such optional ingredients are suitable for oral administration and are compatible with the essential ingredients in the nutritional powders.

Non-limiting examples of such optional ingredients include preservatives, antioxidants, emulsifying agents, buffers, pharmaceutical actives, additional nutrients as described herein, colorants, flavors, thickening agents and stabilizers, and combinations thereof.

The nutritional powders may further comprise vitamins or related nutrients, non-limiting examples of which include vitamin A, vitamin E, vitamin K, thiamine, riboflavin, pyridoxine, vitamin B 12, carotenoids, niacin, folic acid, pantothenic acid, biotin, vitamin C, choline, inositol, salts, and derivatives thereof, and combinations thereof.

The nutritional powders may further comprise minerals, non-limiting examples of which include phosphorus, magnesium, iron, zinc, manganese, copper, sodium, potassium, molybdenum, chromium, selenium, chloride, and combinations thereof.

### Method of Use

The nutritional powders are reconstituted with water or other suitable liquid to provide a nutritional liquid. Such liquids from the powders described herein are useful to provide supplement, primary, or sole sources of nutrition, and or to provide individuals one or more benefits as described herein. In accordance with such methods, the liquids may be administered orally as needed to provide the desired level of nutrition, most typically in the form of one to two servings daily, in one or two or more divided doses daily, e.g., serving sizes typically ranging from about 100 to about 300 ml, including from about 150 to about 250 ml, including from about 190 ml to about 240 ml, wherein each serving contains from about 0.4 to about 3.0 g, including from about 0.75 to about 2.0 g, including about 1.5 g, of calcium HMB per serving.

Such methods are further directed to provide the individual upon administration of such products, most typically after daily use over an extended period of time of from about 1 to about 6 months, including from about 1 to about 3 months, one or more of 1) to support maintenance of lean body mass, 2) to support of strength and or muscle strength, 3) to decrease protein breakdown and damage of muscle cells, and 4) to help with muscle recovery following exercise or other trauma, and 5) to reduce muscle protein breakdown following exercise.

Such methods are also helpful to achieve one or more of 1) to maintain and support lean body mass in elderly with sarcopenia, 2) to provide nutrition to support an active and independent lifestyle in individuals, especially in the elderly, 3) to support recovery of muscle strength, 4) to help rebuild muscle and regain strength, and 5) to improve strength, including muscle strength, and mobility.

### Methods of Manufacture

The nutritional powders may be prepared by any collection of known or otherwise effective techniques, suitable for making and formulating a nutritional powder provided that the techniques include or are modified to include a spray drying step to provide the powder with the requisite spray dried HMB ingredient.

The spray drying step may likewise include any spray drying technique that is known for or otherwise suitable for use in the production of nutritional powders. Many different spray drying methods and techniques are known for use in the nutrition field, all of which are suitable for use in the manufacture of the nutritional powders herein.

One method of preparing the nutritional powder comprises forming an aqueous slurry or liquid comprising HMB and at least one of protein, carbohydrate, and fat, and then spray drying the slurry or liquid to produce a spray dried nutritional powder comprising spray dried HMB and having improved sensory properties. The method may further comprise the step of spray drying, dry mixing, or otherwise adding additional nutritional ingredients, including any one or more of the ingredients described herein, to the spray dried nutritional powder.

The methods of manufacture are preferably formulated with calcium HMB, which is most typically formulated as calcium HMB monohydrate, as the HMB source for use in the methods.

In accordance with the methods of manufacture herein, it is preferred that all of the HMB in the nutritional powder is spray dried as describe herein, and more preferably that from 50% to 100%, most preferably 100%, by weight of the nutritional powder is spray dried as well. It is most preferred therefore, that most or all of the ingredients in the nutritional powder, including all of the HMB in the powder, are spray dried together to form a spray dried nutrition powder wherein 100% of the HMB is therefore spray dried HMB.

Also in accordance with the methods of manufacture herein, a portion of the HMB in the nutritional powder may be spray dried with some or all of at least one of the fat, protein, and carbohydrate in the nutritional powder, including from 20% to 90%, also including from 40% to 90%, and also including from 50% to 80%, by weight of the total HMB as spray dried HMB. In such nutritional powders, a portion of the HMB may be non-spray and thus represent from 10% to 80%, including from 10% to 60%, and also including from 20% to 50%, by weight of the total HMB in the nutritional powder.

The nutritional powders are preferably spray dried to a moisture content of less than about 3.0%, most typically from about 1.0% to about 2.8%, by weight of the finished powder.

### EXAMPLES

The following examples illustrate specific embodiments and/or features of the nutritional powders comprising spray dried HMB. The examples are given solely for the purpose of illustration and are not to be construed as limitations of the present disclosure, as many variations thereof are possible without departing from the spirit and scope of the disclosure. All exemplified amounts are weight percentages based upon the total weight of the composition, unless otherwise specified.

The exemplified compositions are nutritional powders prepared in accordance with the manufacturing methods described herein, such that each exemplified nutritional powders comprising spray dried HMB has minimal or no off odors and is packaged in sealed containers, and then a portion of which is later removed and reconstituted with water to the desired caloric density to form a nutritional liquid containing 1.5 g of calcium HMB monohydrate per 240 ml of reconstituted liquid. The 240ml liquid represents a single serving suitable for oral administration within 48 hours following such constitution.

In general, each of the exemplified compositions can be manufactured by 1) preparing a first liquid slurry (CHO/MIN) comprising water, carbohydrates, minerals, and calcium HMB monohydrate, 2) preparing a second liquid slurry comprising oils. stabilizers, oil soluble vitamins, and protein (PROTEIN/OIL), 3) preparing a third liquid slurry comprising protein an water (PROTEIN/WATER), 4) blending the first and second slurries together, and then blending the resulting slurry with the third slurry, 5) subjecting the resulting blend to homogenization and heat treatment, 6) standardizing the resulting homogenized blend with vitamins, flavors, trace and ultra trace minerals, and other heat sensitive ingredients, and then 7) spray drying the standardized blend to form a spray dried nutritional powder. The spray dried powder is then package and hermetically sealed in appropriate containers suitable for long term storage prior to use, during which the product may be opened and product reconstituted to form a nutritional liquid and administered in accordance with the methods described herein.

### Examples 1-4

Examples 1-4 illustrate nutritional powders of the present disclosure, the ingredients of which are listed in the table below. All ingredient amounts are listed as kilogram per 1000 kilogram batch of product, unless otherwise specified.

| Ingredient | Example 1 | Example 2 | Example 3 | Example 4 |
|---|---|---|---|---|
| Whey Protein Concentrate | 282.051 | 293.892 | 289.892 | 284.892 |
| Calcium Caseinate | 192.308 | 192.308 | 192.308 | 192.308 |
| Maltodextrin | 165.416 | 165.416 | 165.416 | 165.416 |
| Milk Protein Isolate | 138.782 | 138.782 | 138.782 | 138.782 |
| Cocoa | 76.932 | 76.932 | 76.932 | 76.932 |
| Sunflower Oil Creamer | 21.474 | 21.474 | 21.474 | 21.474 |
| Oil Preblend | 19.231 | 19.231 | 19.231 | 19.231 |
| Chocolate Cream | 15.256 | 15.256 | 15.256 | 15.256 |
| Calcium HMB Monohydrate | 13.157 | 1.316 | 5.316 | 10.316 |
| Oat Fiber | 10.897 | 10.897 | 10.897 | 10.897 |
| Tricalcium Phosphate | 8.526 | 8.526 | 8.526 | 8.526 |
| Vitamin Mineral Preblend | 8.462 | 8.462 | 8.462 | 8.462 |
| Dipotassium Phosphate | 8.333 | 8.333 | 8.333 | 8.333 |
| Rich Dark Chocolate | 7.051 | 7.051 | 7.051 | 7.051 |
| Carrageenan | 6.474 | 6.474 | 6.474 | 6.474 |
| Potassium Chloride | 5.128 | 5.128 | 5.128 | 5.128 |
| Salt | 3.205 | 3.205 | 3.205 | 3.205 |
| Xanthan Gum | 3.205 | 3.205 | 3.205 | 3.205 |
| Choline Bitartrate 41% Choline | 2.782 | 2.782 | 2.782 | 2.782 |
| Acesulfame K | 2.718 | 2.718 | 2.718 | 2.718 |
| Vanilla | 1.923 | 1.923 | 1.923 | 1.923 |
| Disodium Phosphate Anhydrous | 1.667 | 1.667 | 1.667 | 1.667 |
| Whey Protein Isolate | 1.282 | 1.282 | 1.282 | 1.282 |
| Beta Carotene 1% | 1.128 | 1.128 | 1.128 | 1.128 |
| Sucralose | 692.3 g | 692.3 | 692.3 | 692.3 |
| Potassium Citrate 38% K | 641.0 g | 641.0 | 641.0 | 641.0 |
| Alpha ketoglutaric Acid | 321.0 g | 321.0 | 321.0 | 321.0 |
| Egg Albumin Powder | 321.0 g | 321.0 | 321.0 | 321.0 |
| L-Glutamine | 321.0 g | 321.0 | 321.0 | 321.0 |
| Taurine | 321.0 g | 321.0 | 321.0 | 321.0 |

### Examples 5-8

Examples 5-8 illustrate nutritional powders of the present disclosure, the ingredients of which are listed in the table below. All ingredient amounts are listed as kg per 1000 kg batch of product, unless otherwise specified.

| Ingredient | Example 5 | Example 6 | Example 7 | Example 8 |
|---|---|---|---|---|
| Whey Protein Concentrate | 280.051 | 272.051 | 262.051 | 232.051 |
| Calcium Caseinate | 192.308 | 192.308 | 192.308 | 192.308 |
| Maltodextrin | 165.416 | 165.416 | 165.416 | 165.416 |
| Milk Protein Isolate | 138.782 | 138.782 | 138.782 | 138.782 |
| Cocoa | 76.932 | 76.932 | 76.932 | 76.932 |
| Sunflower Oil Creamer | 21.474 | 21.474 | 21.474 | 21.474 |
| Oil Preblend | 19.231 | 19.231 | 19.231 | 19.231 |
| Chocolate Cream | 15.256 | 15.256 | 15.256 | 15.256 |
| Calcium HMB Monohydrate | 15.157 | 25.157 | 35.157 | 50.157 |
| Oat Fiber | 10.897 | 10.897 | 10.897 | 10.897 |
| Tricalcium Phosphate | 8.526 | 8.526 | 8.526 | 8.526 |
| Vitamin Mineral Preblend | 8.462 | 8.462 | 8.462 | 8.462 |
| Dipotassium Phosphate | 8.333 | 7.051 | 7.051 | 7.051 |
| Rich Dark Chocolate | 7.051 | 7.051 | 7.051 | 7.051 |
| Carrageenan | 6.474 | 6.474 | 6.474 | 6.474 |
| Potassium Chloride | 5.128 | 5.128 | 5.128 | 5.128 |
| Salt | 3.205 | 3.205 | 3.205 | 3.205 |
| Xanthan Gum | 3.205 | 3.205 | 3.205 | 3.205 |
| Choline Bitartrate 41% Choline | 2.782 | 2.782 | 2.782 | 2.782 |
| Acesulfame K | 2.718 | 2.718 | 2.718 | 2.718 |
| Vanilla | 1.923 | 1.923 | 1.923 | 1.923 |
| Disodium Phosphate Anhydrous | 1.667 | 1.667 | 1.667 | 1.667 |
| Whey Protein Isolate | 1.282 | 1.282 | 1.282 | 1.282 |
| Beta Carotene 1% | 1.128 | 1.128 | 1.128 | 1.128 |
| Sucralose | 692.3g | 692.3 g | 692.3 g | 692.3 g |
| Potassium Citrate 38% K | 641.0 g | 641.0 g | 641.0 g | 641.0 g |
| Alpha ketoglutaric Acid | 321.0 g | 321.0 g | 321.0 g | 321.0 g |
| Egg Albumin Powder | 321.0 g | 321.0 g | 321.0 g | 321.0 g |
| L-Glutamine | 321.0 g | 321.0 g | 321.0 g | 321.0 g |
| Taurine | 321.0 g | 321.0 g | 321.0 g | 321.0 g |

### Examples 9-12

Examples 9-12 illustrate nutritional powders of the present disclosure, the ingredients of which are listed in the table below. All ingredient amounts are listed as kg per 1000 kg batch of product, unless otherwise specified.

| Ingredient | Example 9 | Example 10 | Example 11 | Example 12 |
|---|---|---|---|---|
| Whey Protein Concentrate | 235.051 | 230.051 | 225.051 | 216.051 |
| Calcium Caseinate | 192.308 | 192.308 | 192.308 | 192.308 |
| Maltodextrin | 165.416 | 165.416 | 165.416 | 165.416 |
| Milk Protein Isolate | 138.782 | 138.782 | 138.782 | 138.782 |
| Dutch Cocoa | 76.932 | 76.932 | 76.932 | 76.932 |
| Sunflower Oil Creamer | 21.474 | 21.474 | 21.474 | 21.474 |
| Oil Preblend | 19.231 | 19.231 | 19.231 | 19.231 |
| Chocolate Cream | 15.256 | 15.256 | 15.256 | 15.256 |
| Calcium HMB Monohydrate | 60.157 | 65.157 | 70.157 | 79.157 |
| Oat Fiber | 10.897 | 10.897 | 10.897 | 10.897 |
| Tricalcium Phosphate | 8.526 | 8.526 | 8.526 | 8.526 |
| Vitamin Mineral Preblend | 8.462 | 8.462 | 8.462 | 8.462 |
| Dipotassium Phosphate | 8.333 | 8.333 | 8.333 | 8.333 |
| Rich Dark Chocolate | 7.051 | 7.051 | 7.051 | 7.051 |
| Carrageenan | 6.474 | 6.474 | 6.474 | 6.474 |
| Potassium Chloride | 5.128 | 5.128 | 5.128 | 5.128 |
| Salt | 3.205 | 3.205 | 3.205 | 3.205 |
| Xanthan Gum | 3.205 | 3.205 | 3.205 | 3.205 |
| Choline Bitartrate 41% Choline | 2.782 | 2.782 | 2.782 | 2.782 |
| Acesulfame K | 2.718 | 2.718 | 2.718 | 2.718 |
| Vanilla | 1.923 | 1.923 | 1.923 | 1.923 |
| Disodium Phosphate Anhydrous | 1.667 | 1.667 | 1.667 | 1.667 |
| Whey Protein Isolate | 1.282 | 1.282 | 1.282 | 1.282 |
| Beta Carotene 1% | 1.128 | 1.128 | 1.128 | 1.128 |
| Sucralose | 692.3 g | 692.3 g | 692.3 g | 692.3 g |
| Potassium Citrate 38% K | 641.0 g | 641.0 g | 641.0 g | 641.0 g |
| Alpha ketoglutaric Acid | 321.0 g | 321.0 g | 321.0 g | 321.0 g |
| Egg Albumin Powder | 321.0 g | 321.0 g | 321.0 g | 321.0 g |
| L-Glutamine | 321.0 g | 321.0 g | 321.0 g | 321.0 g |
| Taurine | 321.0 g | 321.0 g | 321.0 g | 321.0 g |

## Claims

1. A nutritional powder comprising HMB and at least one of fat, protein, and carbohydrate, wherein the HMB is spray dried with at least a portion of at least one of the fat, protein and carbohydrate.

2. The nutritional powder of claim 1 wherein the HMB comprises calcium HMB.

3. The nutritional powder of claim 1 wherein the powder comprises from about 0.1% to about 8% by weight of HMB.

4. The nutritional powder of claim 1 wherein the powder comprises from about 5% to about 75% protein, from about 20% to about 90% carbohydrate, and from about 5% to about 75 % fat, all by weight of the nutritional powder.

5. The nutritional powder of claim 1 wherein the powder comprises from about 12% to about 30% protein, from about 30% to about 80% carbohydrate, and from about 10% to about 35 % fat, all by weight of the nutritional powder.

6. The nutritional powder of claim 1 wherein 100% of the nutritional powder is spray dried.

7. The nutritional powder of claim 1 comprising from 0.1 % to 8% calcium HMB, from about 5% to about 75% protein, from about 20% to about 90% carbohydrate, and from about 5% to about 75 % fat, all by weight of the nutritional powder.

8. The nutritional powder of claim 7 wherein the calcium HMB is a monohydrate salt.

9. The nutritional powder of claim 7, wherein all of the nutritional powder is spray dried.

10. The nutritional powder of claim 7 wherein the powder comprises from about 0.4% to about 1.5% by weight of calcium HMB.

11. A method of preparing a nutritional powder comprising spray dried HMB, the method comprising: preparing a liquid slurry comprising HMB and at least one of protein, carbohydrate, and fat; and spray drying the slurry to produce a spray dried nutritional powder comprising spray dried HMB.

12. The method of claim 11 further comprising the step of adding additional ingredients after the spray drying.

13. The method of claim 11 wherein 100% of the HMB present in the nutritional powder is spray dried HMB.

14. The method of claim 11 wherein the HMB comprises calcium HMB.

15. The nutritional powder of claim 1 comprising from 0.1 % to 8% calcium HMB monohydrate, from about 5% to about 75% protein, from about 20% to about 90% carbohydrate, and from about 5% to about 75 % fat, all by weight of the nutritional powder, wherein the calcium HMB is spray dried with the fat, protein and carbohydrate in the nutritional powder.

## Patentansprüche

1. Ein Nahrungspulver, das HMB und mindestens eines von Fett, Protein und Kohlenhydrat umfasst, worin das HMB mit mindestens einem Teil von mindestens einem des Fetts, des Proteins und des Kohlenhydrats sprühgetrocknet ist.

2. Das Nahrungspulver gemäß Anspruch 1, worin das HMB Kalzium-HMB umfasst.

3. Das Nahrungspulver gemäß Anspruch 1, worin das Pulver von ungefähr 0,1 Gewichtsprozent bis ungefähr 8 Gewichtsprozent HMB umfasst.

4. Das Nahrungspulver gemäß Anspruch 1, worin das Pulver von ungefähr 5% bis ungefähr 75% Protein, von ungefähr 20% bis ungefähr 90% Kohlenhydrat und von ungefähr 5% bis ungefähr 75% Fett, alles nach Gewicht des Nahrungspulvers, umfasst.

5. Das Nahrungspulver gemäß Anspruch 1, worin das Pulver von ungefähr 12% bis ungefähr 30% Protein, von ungefähr 30% bis ungefähr 80% Kohlenhydrat und von ungefähr 10% bis ungefähr 35% Fett, alles nach Gewicht des Nahrungspulvers, umfasst.

6. Das Nahrungspulver gemäß Anspruch 1, worin 100% des Nahrungspulvers sprühgetrocknet ist.

7. Das Nahrungspulver gemäß Anspruch 1, das von 0,1% bis 8% Kalzium-HMB, von ungefähr 5% bis ungefähr 75% Protein, von ungefähr 20% bis ungefähr 90% Kohlenhydrat und von ungefähr 5% bis ungefähr 75% Fett, alles nach Gewicht des Nahrungspulvers, umfasst.

8. Das Nahrungspulver gemäß Anspruch 7, worin das Kalzium-HMB ein Monohydratsalz ist.

9. Das Nahrungspulver gemäß Anspruch 7, worin das gesamte Nahrungspulver sprühgetrocknet ist.

10. Das Nahrungspulver gemäß Anspruch 7, worin das Pulver von ungefähr 0,4 Gewichtsprozent bis ungefähr 1,5 Gewichtsprozent Kalzium-HMB umfasst.

11. Ein Verfahren zur Herstellung eines Nahrungspulvers, das sprühgetrocknetes HMB umfasst, wobei das Verfahren Folgendes umfasst: Herstellung einer flüssigen Aufschlämmung, die HMB und mindestens eines von Protein, Kohlenhydrat und Fett umfasst, und Sprühtrocknung der Aufschlämmung, um ein sprühgetrocknetes Nahrungspulver herzustellen, das sprühgetrocknetes HMB umfasst.

12. Das Verfahren gemäß Anspruch 11, das weiter den Schritt der Hinzugabe zusätzlicher Ingredienzen nach der Sprühtrocknung umfasst.

13. Das Verfahren gemäß Anspruch 11, worin 100% des im Nahrungspulver vorhandenen HMBs sprühgetrocknetes HMB sind.

14. Das Verfahren gemäß Anspruch 11, worin das HMB Kalzium-HMB umfasst.

15. Das Nahrungspulver gemäß Anspruch 1, das Folgendes umfasst: von 0,1% bis 8% Kalzium-HMB-Monohydrat, von ungefähr 5% bis ungefähr 75% Protein, von ungefähr 20% bis ungefähr 90% Kohlenhydrat und von ungefähr 5% bis ungefähr 75% Fett, alles nach Gewicht des Nahrungspulvers, worin das Kalzium-HMB mit dem Fett, Protein und Kohlenhydrat im Nahrungspulver sprühgetrocknet ist.

## Revendications

1. Poudre nutritionnelle comprenant du HMB et au moins l'un de graisse, protéine et glucide, où le HMB est séché par pulvérisation avec au moins une partie d'au moins l'un de la graisse, la protéine et le glucide.

2. Poudre nutritionnelle selon la revendication 1 où le HMB comprend du HMB de calcium.

3. Poudre nutritionnelle selon la revendication 1 où la poudre comprend d'environ 0,1 % à environ 8 % en poids de HMB.

4. Poudre nutritionnelle selon la revendication 1 où la poudre comprend d'environ 5 % à environ 75 % de protéine, d'environ 20 % à environ 90 % de glucide et d'environ 5 % à environ 75 % de graisse, tous en poids de la poudre nutritionnelle.

5. Poudre nutritionnelle selon la revendication 1 où la poudre comprend d'environ 12 % à environ 30 % de protéine, d'environ 30 % à environ 80 % de glucide et d'environ 10 % à environ 35 % de graisse, tous en poids de la poudre nutritionnelle.

6. Poudre nutritionnelle selon la revendication 1 où 100 % de la poudre nutritionnelle est séché par pulvérisation.

7. Poudre nutritionnelle selon la revendication 1 comprenant de 0,1 % à 8 % de HMB de calcium, d'environ 5 % à environ 75 % de protéine, d'environ 20 % à environ 90 % de glucide et d'environ 5 % à environ 75 % de graisse, tous en poids de la poudre nutritionnelle.

8. Poudre nutritionnelle selon la revendication 7 où le HMB de calcium est un sel monohydraté.

9. Poudre nutritionnelle selon la revendication 7 où toute la poudre nutritionnelle est séchée par pulvérisation.

10. Poudre nutritionnelle selon la revendication 7 où la poudre comprend d'environ 0,4 % à environ 1,5 % en poids de HMB de calcium.

11. Procédé de préparation d'une poudre nutritionnelle comprenant du HMB séché par pulvérisation, le procédé comprenant : la préparation d'une suspension liquide comprenant du HMB et au moins l'un de protéine, glucide et graisse ; et le séchage par pulvérisation de la suspension pour produire une poudre nutritionnelle séchée par pulvérisation comprenant du HMB séché par pulvérisation.

12. Procédé selon la revendication 11 comprenant en outre l'étape d'addition d'ingrédients supplémentaires après le séchage par pulvérisation.

13. Procédé selon la revendication 11 où 100 % du HMB présent dans la poudre nutritionnelle est du HMB séché par pulvérisation.

14. Procédé selon la revendication 11 où le HMB comprend du HMB de calcium.

15. Poudre nutritionnelle selon la revendication 1 comprenant de 0,1 % à 8 % de HMB de calcium monohydraté, d'environ 5 % à environ 75 % de protéine, d'environ 20 % à environ 90 % de glucide et d'environ 5 % à environ 75 % de graisse, tous en poids de la poudre nutritionnelle, où le HMB de calcium est séché par pulvérisation avec la graisse, la protéine et le glucide dans la poudre nutritionnelle.
